# FASCICULE DE BREVET EUROPEEN

(11) **EP 0 834 316 B1**
(45) Date de publication et mention de la délivrance du brevet: **02.05.2002**
(21) Numéro de dépôt: 97402320.2
(22) Date de dépôt: 03.10.1997
(51) Int. Cl.: A61K 31/505, A61P 25/16, A61P 25/18

(54) **Utilisation de la sulbutiamine pour l'obtention de compositions pharmaceutiques utiles dans le traitement de certains troubles psychomoteurs et psycho-intellectuels**
Verwendung von Sulbutiamin zur Behandlung von psychomotorischen oder psychointellektuellen Beschwerden
Use of sulbutiamine for the treatment of psychomotor or psycho-intellectual problems

(30) Priorité: 04.10.1996 FR 9612112
(43) Date de publication de la demande: 08.04.1998
(73) Titulaire: LES LABORATOIRES SERVIER, 92200 Neuilly sur Seine (FR)
(72) Inventeur: Ollat, Hélène, 77540 Nesles la Gilberde (FR); Le Ridant, Alain, 92200 Neuilly sur Seine (FR); Perret, Laurent, 75006 Paris (FR)

(56) Documents cités:
- L. CROCQ ET AL.: "Etude de l'activité de l'arcalion dans les syndromes dépressifs névrotiques et réactionnels." PSYHOL. MED., vol. 10, no. 4, 1978, pages 797-813, XP000675576
- O. VAN REETH ET AL.: "Age-related changes in the hamster's circadian system partially reversed by treatment with sulbutiamine, a Vit B-1 related compound." BIOL. RYTHM RES., vol. 25, no. 4, 1994, pages 477-479, XP000675634
- B. CHAUVOT: "L'arcalion 200 en prescription psychiatrique et anti-alcoolique ambulatoire et institutionelle." PSYCHOL. MED., vol. 12, no. 9, 1980, pages 1985-1991, XP000675574
- P. BUGARD: " tude de l'arcalion 200." GAZ. MED. FR., vol. 86, no. 6, 1979, pages 569-598, XP000675575

## Description

La présente invention concerne l'utilisation de la sulbutiamine pour l'obtention de compositions pharmaceutiques utiles au traitement de certains troubles psychomoteurs et psycho-intellectuels, caractérisés par le retard, le ralentissement et la dépression des réponses comportementales et intellectuelles exigeant la mobilisation stratégique des percepts et des concepts mentaux. Ces troubles sont en particulier observés chez les malades parkinsoniens et schizophrènes déficitaires.

La sulbutiamine est un principe actif déjà connu et décrit dans la littérature. Le brevet spécial de médicament 5921 M a décrit ce produit comme agent ayant l'activité de la vitamine B₁, susceptible d'amener un taux sanguin en vitamine B₁ élevé et capable d'exercer des effets vis-à-vis de tous les symptômes de l'avitaminose B₁.

Le BSM 5921 M mentionne également qu'à ces fins thérapeutiques, le produit est utilisé sous forme de tablettes renfermant de 5 à 50 mg de produit par prise unitaire.

Enfin, le brevet belge BE 845.260 décrit les propriétés psychoanaleptiques de la sulbutiamine, qui permettent d'antagoniser les effets neuro-dépresseurs des sédatifs ou des neuroleptiques.

Le Vidal, quant à lui, préconise l'utilisation de la sulbutiamine pour le traitement de certains états d'inhibition physique ou psychique avec baisse d'activité et apathie.

La demanderesse a présentement démontré que l'administration de sulbutiamine permet la récupération des troubles psychomoteurs et psycho-intellectuels décrits au premier paragraphe, chez les malades parkinsoniens et des schizophrènes déficitaires.

Dans les différentes études menées chez ces malades, la sulbutiamine a permis dans tous les cas d'éviter le ralentissement psychomoteur, l'inhibition à l'action, les troubles de la stratégie intellectuelle, ainsi que les troubles des fonctions exécutives.

Les compositions pharmaceutiques utilisées selon l'invention renferment la sulbutiamine, seule ou en combinaison avec un ou plusieurs véhicules inertes, non toxiques, pharmaceutiquement acceptables.

Les médicaments destinés au traitement des troubles psychomoteurs et intellectuels chez ces sujets, obtenus en utilisant selon l'invention la sulbutiamine, seront présentés sous des formes pharmaceutiques convenant pour l'administration par voie orale, parentérale, transcutanée, nasale, rectale, perlinguale notamment les comprimés, les comprimés sublinguaux, les glossettes, les gélules, les capsules, les tablettes, les suppositoires, les patchs transdermiques, les patchs buccaux, etc...

La posologie varie selon l'âge et le poids du patient, la voie d'administration, la nature de l'indication thérapeutique et des traitements associés, et s'échelonne de 400 mg à 800 mg par jour par voie orale.

### ETUDES CLINIQUES

### EXEMPLE 1: Effets de la sulbutiamine sur le ralentissement cognitif, objectif et subjectif, et sur la sensation de fatigue des malades parkinsoniens

L'étude a été réalisée sur une population de malades présenant une maladie de Parkinson idiopathique, traités par L-Dopa et/ou agonistes dopaminergiques, stables à l'entrée dans l'étude, sans fluctuations majeures de l'état moteur et sans signes démentiels.

Ces patients ont été répartis en 2 groupes parallèles et ont été traités en double aveugle pendant 8 semaines, soit par de la sulbutiamine, soit par un placebo, les traitements étant attribués selon un code de randomisation.

Grâce aux tests suivants, on a évalué les effets du traitement sur :
1- les fonctions cognitives, l'attention, le ralentissement verbal, idéïque et moteur :
   - test des 15 objets,
   - test de Stroop,
   - échelle MADRS (difficultés de concentration),
   - test de fluence verbale,
   - Trail Making Test.
2- les fonctions exécutives :
   - Odd Man Out Test,
   - Wais-R.
3- les fonctions mnsésiques :
   - test de Grôber et Buschke.
4- la sensation de fatigue :
   - autoévaluation de la fatigue.
5- l'humeur :
   - échelle MADRS (émoussement émotionnel, baisse d'activité).

Il a été observé chez les malades parkinsoniens, traités par sulbutiamine, une amélioration des fonctions cognitives, exécutives et mnsésiques, avec diminution de la sensation de fatigue.

### EXEMPLE 2 : Effets de la sulbutiamine sur les symptômes d'inhibition des schizophrènes déficitaires traités par un neuroleptique

L'étude a porté sur des patients schizophrènes déficitaires traités en ambulatoire par un neuroleptique.

Ces patients ont été répartis en 2 groupes parallèles, puis traités en double aveugle pendant 12 semaines, soit par de la sulbutiamine, soit par un placebo, les traitements étant attribués selon un code de randomisation.

On a évalué le traitement sur :
1- les fonctions cognitives :
   - échelle PANSS,
   - Trail Making Test,
   - test de Stroop,
   - échelle visuelle analogique de Norris,
   - échelle MADRS (troubles de concentration, baisse d'activité).
2- les fonctions exécutives :
   - Wisconsin Card Sorting Test.
3- les fonctions mnésiques :
   - test de Gröber et Buschke.
4- les signes négatifs de la schizophrénie :
   - échelle PANSS.
5- la qualité de la vie :
   - échelle de qualité de vie de Heinrichs.

Il a été observé une régression des signes de la schizophrénie dans le groupe traité par sulbutiamine, avec parallèlement une amélioration de la qualité de vie.

## Revendications

1. Utilisation de la sulbutiamine pour l'obtention de compositions pharmaceutiques utiles dans le traitement de certains troubles psychomoteurs et psycho-intellectuels observés chez les malades parkinsoniens et schizophrènes déficitaires.

2. Utilisation selon la revendication 1 de la sulbutiamine pour l'obtention de compositions pharmaceutiques utiles dans le traitement des troubles psychomoteurs et psycho-intellectuels **caractérisés par** le retard, le ralentissement et la depression des réponses comportementales et intellectuelles exigeant la mobilisation stratégique des percepts et des concepts mentaux.

3. Utilisation selon la revendication 2 de la sulbutiamine pour l'obtention de compositions pharmaceutiques renfermant ce principe actif, seul ou en combinaison avec un ou plusieurs véhicules inertes, non toxiques, pharmaceutiquement acceptables.

## Patentansprüche

1. Verwendung von Sulbutiamin für die Herstellung von pharmazeutischen Zubereitungen, die nützlich sind bei der Behandlung bestimmter psychomotorischer und psychointellektueller Störungen, die man bei Parkinson-Erkrankten und Erkrankten, die an defizitärer Schizophrenie leiden, beobachtet.

2. Verwendung nach Anspruch 1 von Sulbutiamin für die Herstellung von pharmazeutischen Zubereitungen, die nützlich sind bei der Behandlung von psychomotorischen und psychointellektuellen Störungen, die **gekennzeichnet sind durch** die Verzögerung, die Verlangsamung und die Unterdrückung von verhaltensmäßigen und intellektuellen Antworten, welche die strategische Mobilisierung von mentalen Erkenntnissen und Konzepten erforderlich machen.

3. Verwendung nach Anspruch 2 von Sulbutiamin für die Herstellung von pharmazeutischen Zubereitungen, die diesen Wirkstoff alleine oder in Kombination mit einem oder mehreren inerten, nicht toxischen, pharmazeutisch annehmbaren Trägermaterialien enthalten.

## Claims

1. Use of sulbutiamine in obtaining pharmaceutical compositions for use in the treatment of certain psychomotor and psycho-intellectual disorders observed in parkinsonian patients and deficit-type schizophrenia patients.

2. Use, according to claim 1, of sulbutiamine in obtaining pharmaceutical compositions for use in the treatment of psychomotor and psycho-intellectual disorders **characterised by** delay, slowing and depression of behavioural and intellectual responses requiring strategic mobilisation of mental concepts and percepts.

3. Use, according to claim 2, of sulbutiamine in obtaining pharmaceutical compositions comprising that active ingredient on its own or in combination with one or more pharmaceutically acceptable, inert, non-toxic carriers.
